(19) Europäisches Patentamt
European Patent Office
Office européen des brevets

(11) Veröffentlichungsnummer : **0 161 501**
**B1**

(12) **EUROPÄISCHE PATENTSCHRIFT**

(45) Veröffentlichungstag der Patentschrift : **15.03.89**

(51) Int. Cl.[4] : **A 61 K 31/47**, A 61 K 45/06 // (A61K31/47, 31:415)

(21) Anmeldenummer : **85104490.9**

(22) Anmeldetag : **12.04.85**

(54) **Antiulcermittel zur Behandlung von Geschwüren.**

(30) Priorität : **13.04.84 BG 65090/84**

(43) Veröffentlichungstag der Anmeldung : **21.11.85 Patentblatt 85/47**

(45) Bekanntmachung des Hinweises auf die Patenterteilung : **15.03.89 Patentblatt 89/11**

(84) Benannte Vertragsstaaten : **CH DE FR GB IT LI SE**

(56) Entgegenhaltungen :
**GB–A– 1 565 966**
**US–A– 3 950 353**
**CHEMICAL ABSTRACTS, Band 50, Nr. 13, 10. Juli 1956, Spalte 9623b, Columbus, Ohio, US; E. LINDNER: "The action of a pyrazolone derivative on smooth-muscled organs" & ARZNEIMITTEL-FORSCH. 6, 124-127, 1956**
**Arzneimittel, 1972, Bd. I, p. 157, The Merck Index, 10 ed., 6515**
**Die Akte enthält technische Angaben, die nach dem Eingang der Anmeldung eingereicht wurden und die nicht in dieser Patentschrift enthalten sind.**

(73) Patentinhaber : **T P O "PHARMACHIM"**
**Iliensko Chaussée 16**
**Sofia (BG)**

(72) Erfinder : **Ivanova, Nedyalka Stoyanova, Dr.**
**11, Traytsho Kostov Str.**
**Sofia (BG)**
Erfinder : **Ivanov, Tshavdar Borissov**
**Komplex Mladost, Block 46-4**
**Sofia (BG)**
Erfinder : **Dryanska, Margarita Dimitrova**
**11, Dzerjinski-Str.**
**Sofia (BG)**
Erfinder : **Zabunova, Orhideya Bontsheva**
**Komplex Mladost-2, Bl. 215-D**
**Sofia (BG)**
Erfinder : **Dalieva, Lilyana Dimitrova, Dr.**
**Block 20, Persenk Str.**
**Sofia (BG)**
Erfinder : **Nikolova, Milka Petrova**
**3, Paiko Daskalov Str.**
**Sofia (BG)**
Erfinder : **Berova, Nikolina Dimitrova**
**Komplex Krasno Selo, Bl. 194-B**
**Sofia (BG)**
Erfinder : **Rakovska, Rossitza Stefanova**
**Block 37, Boul. N. Vapzarov**
**Sofia (BG)**
Erfinder : **Stoyanova, Maria Stoyanova**
**Komplex Tolstoy II Block 9**
**Sofia (BG)**
Erfinder : **Mihaylova, Sascha Radkova**
**116-B, Boul. G. Dimitrov**
**Sofia (BG)**
Erfinder : **Luna, Milka Aron**
**86, Positano Str.**
**Sofia (BG)**

**EP 0 161 501 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Erfinder: **Nissimov, Jossif Nissim**
**37, Sofronii Str.**
**Sofia (BG)**
Erfinder: **Vitkova, Snejana Georgieva**
**Block 252, N. Kamenov Str.**
**Sofia (BG)**
Erfinder: **Matov, Vladimir Konstantinov**
**45, Ivan Assen-II Str.**
**Sofia (BG)**
Erfinder: **Dimtrov, Boris Krumov**
**39, Boul. 9-ti Septemvri**
**Sofia (BG)**
Erfinder: **Metshkov, Grigor Mintshev**
**Block 9, Boul. St. Lepoev**
**Sofia (BG)**

(74) Vertreter: **von Füner, Alexander, Dr. et al**
**Patentanwälte v. Füner, Ebbinghaus, Finck Mariahilfplatz 2 & 3**
**D-8000 München 90 (DE)**

**Beschreibung**

Die Erfindung betrifft ein Mittel zur Behandlung von Magen- und Zwölffingerdarmgeschwüren, zur Prophylaxe bei schweren Brandwunden, zur Behandlung bei unterschiedlichen schockartigen Zuständen und zur Verhinderung von Streßgeschwüren.

Es sind Antagonisten der Histamin $H_2$-Rezeptoren bekannt, wie Cimetidin, N-Cyano-N'-methyl-N''-[2-(4-methylimidazolyl) methyl-thio]-ethylguanidin (US-A-3 950 353) und andere Guanidinderivate, wie Ranitidin (N-(2-[(5-Dimethylaminomethyl-2-furanyl)-methyl] thioethyl)-N-methyl-2-nitro-1,1-ethendiamin) (GB-A-1 565 966), welche die Magensekretion bei Menschen und Tieren inhibieren. Sie zeichnen sich als wirksam beim Unterdrücken der histamin- und pentagastrinsimulierten Sekretion aus.

Die wesentlichsten unerwünschten Nebenerscheinungen des Cimetidins sind Magen-Zwölffingerdarm-Schleimhauterosionen, Magenkrebs, Perforation, Pankreasentzündung, Bradykardie, Leukopenie, Agranulozytose, Haut-Hypersensibilität, autoimmune hämolytische Hepatitis, Hyperglykämie, Ästhenie und Muskelschmerzen (Kato, H. et al., Arch. Pharmacol. et Therapie, 1981, 249 (2), S. 247-256).

Es ist bekannt, daß 4-Phenyl-2-methyl-8-amino-1,2,3,4-tetrahydroisochinolinsaures Maleat der Formel 1 unter der Bezeichnung Nomifensin eine antidepressive Wirkung aufweist.

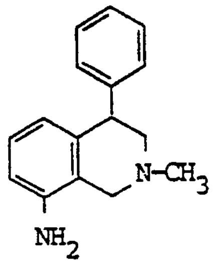

Bekannt ist auch, daß die Verbindung 4-(β-Piperidino-ethoxy)-2'-methoxycarbonyl-benzophenon (Novalgin) eine spasmolytische und analgetische Wirkung aufweist (C.A., 50, 9623b, E. Lindner, Arzn. Forsch., 1956, 6, 124-7).

Aufgabe der Erfindung war es nun, ein Mittel zur Behandlung bzw. gegen die Bildung von Geschwüren (Antiulcermittel) mit einer verstärkten Wirkung gegen Geschwüre zu erarbeiten, das eine minimale Toxizität und schwach ausgeprägte Nebenerscheinungen aufweist.

Diese Aufgabe wird durch die Anwendung eines Mittels auf der Basis von Histamin $H_2$-Blocker gelöst, insbesondere N-Cyano-N'-methyl-N''-[2-(4-methyl-5-imidazolyl)-methylthio]-ethyl-guanidin (Cimetidin) und (N-(2-[(5-Dimethylaminoethyl-2-furanyl)-methyl]-thioethyl)-N-methyl-2-nitro-1,1-ethendiamin) (Ranitidin), welches als Antidepressant 4-Phenyl-2-methyl-8-amino-1,2,3,4-tetrahydroisochinolin und/oder dessen optisch aktive Isomere oder dessen physiologisch verträglichen Salze und gegebenenfalls ein krampflösendes Mittel enthält, wie 2'-Methoxycarbonyl-4-(β-piperidino-ethoxy) benzophenon), wobei das Verhältnis des Histamin $H_2$-Blockers zum Antidepressant zwischen 160 : 1 und 2 : 1 liegt und das Verhältnis der Summe der ersten beiden zum krampflösenden Mittel von 425 : 1 bis 2,2 : 1 beträgt.

Es wurde festgestellt, daß das racemische Nomifensin und das Cimetidin eine äußerst günstige Wechselwirkung bei ihrer gleichzeitigen Einführung besitzen, um den Ulcerindex zu vermindern im Vergleich zu der Anwendung von Cimetidin allein. Das Kombinieren der Komponenten im erfindungsgemäßen Mittel führt zu einem Synergismus.

Der Antiulcereffekt der Kombination wird noch weiter erhöht bei der Verwendung des optisch aktiven (+)-Nomifensinisomers, da das (—)-Nomifensin nicht aktiv ist.

Durch die Versuche wurde festgestellt, daß der Antiulcereffekt des Cimetidins 20 mg/kg und /+/ Nomifensins 1 mg/kg dem Antiulcereffekt des Cimetidin 20 mg/kg und razemischen Nomifensins 2 mg/kg gleich ist, d. h. daß das /+/ Nomifensin um das Doppelte aktiver im Vergleich zum Razemat ist.

Einmalige Cimetidin-Dosen können von 50 bis 400 mg betragen, die des /+/ Nomifensins von 2,5 bis 25 mg und eventuell des Novalgins von 1 bis 5 mg. Die 24-stündigen Dosen betragen von 150 bis 2 400 mg für das Cimetidin für das /+/ Nomifensin sind sie von 15 bis 150 mg, und für das Novalgin$^{(R)}$ betragen sie 3 bis 30 mg, welche auf drei- bis sechsmalige Einnahmen verteilt werden.

Die Vorteile des erfindungsgemäßen Antiulcermittels bestehen in der stark ausgeprägten Gegengeschwürwirkung, der Verminderung der Dosis des $H_2$ Blockers, insbesondere des Cimetidins, und der Potenzierung seines Antiulcereffekts, wobei gleichzeitig seine Nebenerscheinungen vermindert werden. Das kombinierte Präparat kann zur Behandlung von Magen- und Zwölffingerdarmgeschwüren, bei durch Streß verursachten Geschwüren und prophylaktisch bei Streßzuständen angewandt werden, um die Bildung eines Geschwürs zu verhindern, bei denen das Cimetidin nicht effektiv ist.

Es ist bevorzugt, das erfindungsgemäße Mittel in den Organismus in Form fertiger Arzneimittel einzuführen. Die Kombination ist für die Formulierung fertiger pharmazeutischer Zusammensetzungen und Präparate geeignet. Die fertigen Arzneimittel können die aktiven Stoffe oder deren pharmazeutisch anwendbare Salze eventuell in einem Gemisch mit den üblichen Hilfsmitteln und Trägern enthalten.

Das erfindungsgemäße Antiulcermittel kann in Filmtabletten oder in Kapseln verabreicht werden.

Beispiel 1

Pharmakologisches Test-Experimentalmodell von Wasserimmersions-Streßgeschwüren. Die Befunde von Untersuchungen sind in den beiliegenden Tabellen 1 bis 4 veranschaulicht.

Die Versuche wurden an 385 weißen Ratten der Wistarrasse beider Geschlechter nach einem bekannten, nur unwesentlich modifizierten Verfahren (1) gemäß C.A., 50, 9623b, E. Lindner, Arzn, Forsch., 1956, 6, 124-7, durchgeführt. 18 Stunden vor dem Versuch wird das Füttern der Tiere eingestellt, jedoch mit freiem Zutritt zu Wasser. Die erfindungsgemäße Kombination wurde oral verabreicht, wonach die Tiere auf dem Rücken auf einer Unterlage unbeweglich angebunden wurden, so daß sie voneinander isoliert waren. Dann wurden sie in auf 23 °C thermoreguliertes Wasser gebracht. Nach fünf Stunden wurden die Tiere getötet und ihr Magen zur Untersuchung herausgenommen.

Aus Tabelle 1 geht hervor, daß 1 mg/kg Cimetidin und 0,1 mg/kg razemisches /±/ Nomifensin den Ulcerindex von 47,6 bei den Kontrolltieren auf 31,6 bei den behandelten unterdrücken, wobei der Prozentsatz der Unterdrückung 33,6 beträgt. Die Erhöhung der Dosen auf 10 mg Cimetidin und 1 mg/kg /±/ Nomifensin führt zur Erhöhung des Antiulcereffekts. Der Prozentsatz der Unterdrückung des Ulcerindex beträgt 51,6. Der größte Antiulcereffekt wird beim Kombinieren von 50 mg/kg Cimetidin und 9 mg/kg razemischem Nomifensin beobachtet. Der Ulcerindex von 30 bei den Kontrolltieren vermindert sich auf 0,73 bei den mit dem erfindungsgemäßen Präparat behandelten Tieren, und der Prozentsatz der Unterdrückung des Ulcerindex beträgt 97,6.

Wie aus Tabelle 2 hervorgeht, vermindert sich beim Kombinieren der drei Präparate Cimetidin 20 mg/kg, /±/ Nomifensin 1 mg/kg und 0,250 mg/kg Novalgin der Ulcerindex von 53,3 bei Kontrolltieren auf 8,7 bei den behandelten, wobei der Prozentsatz der Unterdrückung 83,7 beträgt, d. h. etwa so viel wie beim Kombinieren des Cimetidins 20 mg/kg und /±/ Nomifensins 3 mg/kg. Die Erhöhung der Dosis des /±/ Nomifensin auf 2 mg/kg führt zur Erhöhung des Antiulcereffekts der Kombination (Tabelle 3).

Der Antiulcereffekt wurde sowohl bei Männchen als auch bei den Rattenweibchen untersucht. Der Ulcerindex von 58,2 bei den Kontrolltieren vermindert sich auf 2,5 bei den behandelten Rattenmännchen, und der Prozentsatz der Unterdrückung beträgt 95,7. Aus der Tabelle geht ebenso hervor, daß das /±/ Nomifensin (— 76,3 %) den größten Antiulcereffekt aufweist und das Zymetidin einen schwächeren (— 52,9 %). Bei den Rattenweibchen ist der Effekt noch stärker ausgeprägt (Tabelle 3). Der Ulcerindex bei den Kontrolltieren vermindert sich von 42,9 auf 0,4 bei den behandelten Tieren, und der Prozentsatz der Unterdrückung der Geschwürbildung beträgt 99 bei den behandelten Tieren, d. h., daß der Antiulcereffekt der Kombination bei diesem Verhältnis mit dem des Cimetidins 50 mg/kg und des /±/ Nomifensins 9 mg/kg gleich ist (Tabelle 1).

Das Austauschen des razemischen Nomifensins in der Kombination durch das optisch aktive /+/ Isomer hat eine um das Doppelte höhere Antiulcer-Aktivität gezeigt (Tabelle 4). Das heißt, daß der Antiulcereffekt des Cimetidins 20 mg/kg und des /+/ Nomifensins 1 mg/kg dem Antiulcereffekt des Cimetidin 20 mg/kg und des /±/ Nomifensins 2 mg/kg gleich ist.

Beispiel 2

A. Filmtabletten

| | |
|---|---|
| Cimetidin | 0,1000 g |
| /+/ Nomifensin | 0,010 g |
| Novalgin(R) | 0,002 g |
| Weizenstärke | 0,043 bis 0,048 g |
| Luviskol A64 | 0,003 bis 0,0035 g |
| Mikrokristallzellulose | 0,058 bis 0,062 g |
| Talk | 0,007 bis 0,008 g |
| Polyvinyl-Pyrrolidon K25 | 0,005 bis 0,006 g |
| Magnesiumstearat | 0,002 bis 0,003 g |
| Aerosil 200 | 0,002 bis 0,004 g |
| Lacküberzug | 0,003 bis 0,005 g |

B. Filmtabletten

| | |
|---|---|
| Cimetidin | 0,100 g |
| /+/ Nomifensin | 0,010 g |
| Weizenstärke | 0,055 bis 0,062 g |
| Luviskol A64 | 0,002 bis 0,004 g |
| Mikrokristallzellulose | 0,055 bis 0,065 g |
| Talk | 0,007 bis 0,008 g |
| Polyvinyl-Pyrrolidon K25 | 0,004 bis 0,006 g |

| | |
|---|---|
| Magnesiumstearat | 0,002 bis 0,003 g |
| Aerosil 200 | 0,002 bis 0,004 g |
| Lacküberzug | 0,003 bis 0,005 g |

C. Kapseln

| | |
|---|---|
| Cimetidin | 0,100 g |
| /+/ Nomifensin | 0,010 g |
| Weizenstärke | 0,040 bis 0,060 g |
| Mikrokristallzellulose | 0,055 bis 0,065 g |
| Talk | 0,007 bis 0,008 g |
| Magnesiumstearat | 0,002 bis 0,003 g |
| Aerosil 200 | 0,002 bis 0,004 g |

D. Kapseln

| | |
|---|---|
| Cimetidin | 0,100 g |
| /+/ Nomifensin | 0,010 g |
| Novalgin(R) | 0,002 g |
| Weizenstärke | 0,040 bis 0,060 g |
| Mikrokristallzellulose | 0,055 bis 0,065 g |
| Talk | 0,007 bis 0,008 g |
| Magnesiumstearat | 0,002 bis 0,003 g |
| Aerosil 200 | 0,002 bis 0,004 g |

(Siehe Tabellen Seite 5 ff.)

Wirkung der Kombination von Cimetidin und razemischem Nomifensin auf ein Modell der Wasser-Immersions-Stressgeschwüre

Tabelle 1

| Eingeführter Stoff in Dosis mg/kg | Kontrolle Ulcerindex | Cimetidin | | Nomifensin | | Cimetidin und Nomifensin | |
|---|---|---|---|---|---|---|---|
| | | Ulcerindex | % der Unterdrückung des Ulcerindex | Ulcerindex | % der Unterdrückung des Ulcerindex | Ulcerindex | % der Unterdrückung des Ulcerindex |
| 1 | 2 | 3 | 4 | 5 | 6 | 7 | 8 |
| Cimetidin 1 mg/kg und Nomifensin 0,1 mg/kg | 47,6 | — | — | — | — | 31,6 | −33,6 |
| Cimetidin 10 mg/kg Nomifensin 1 mg/kg | 43,4 | 39,8 | −8,3 | 21,8 | −49,8 $p<0,05$ | 21 | −51,6 $p<0,05$ |
| Cimetidin 20 mg/kg Nomifensin 3 mg/kg | 30 | — | — | — | — | 3,5 | −88,3 $p<0,001$ |
| Cimetidin 50 mg/kg | 30 | — | — | — | — | 0,73 | −97,6 $p<0,001$ |

Wirkung der Kombination von Cimetidin, Nomifensin Racemat und Novalgin(R) auf ein Experimentalmodell von Wasser-Immersions-Stressgeschwüren

Tabelle 2

| Eingeführter Stoff mg/kg | Kontrolle Ulcerindex | Cimetidin | | Nomifensin | | Novalgin(R) | | Cimetidin, Nomifensin, Novalgin(R) | |
|---|---|---|---|---|---|---|---|---|---|
| | | Ulcer-index | % der Unter-drückung des Ulcerindex | Ulcer-index | % der Unter-drückung des Ulcerindex | Ulcer-index | % der Unter-druckung des Ulcerindex | Ulcer-index | % der Unter-drückung des Ulcerindex |
| 1 | 2 | 3 | 4 | 5 | 6 | 7 | 8 | 9 | 10 |
| Cimetidin 20 mg/kg Nomifensin 1 mg/kg Novalgin(R) 25 mg/kg | 53,3 | — | — | — | — | — | — | 8,7 | −83,7 $p<0{,}001$ |

Wirkung der Kombination von Cimetidin, Nomifensin Racemat, Novalgin(R) auf ein Experimentalmodell von Wasser-Immersions-Geschwüren

Tabelle 3

| Eingeführter Stoff Dosis mg/kg | Kontrolle Ulcerindex | Cimetidin | | Nomifensin | | Novalgin(R) | | Cimetidin, Nomifensin, Novalgin(R) | |
|---|---|---|---|---|---|---|---|---|---|
| | | Ulcer-index | % der Unterdrückung des Ulcerindex | Ulcer-index | % der Unterdrückung des Ulcerindex | Ulcer-index | % der Unterdruckung des Ulcerindex | Ulcer-index | % der Unterdrückung des Ulcerindex |
| 1 | 2 | 3 | 4 | 5 | 6 | 7 | 8 | 9 | 10 |
| | | | | Rattenmännchen | | | | | |
| Cimetidin 20 mg/kg Nomifensin 2 mg/kg Novalgin(R) 0,25 mg/kg | 58,2 | 27,4 | −52,9 $\rho<0,01$ | 13,8 | −76,3 $\rho<0,001$ | 39,1 | −32,8 | 2,5 | −95,7 $\rho<0,01$ |
| | | | | Rattenweibchen | | | | | |
| Cimetidin 20 mg/kg Nomifensin 2 mg/kg Novalgin(R) 0,25 mg/kg | 42,9 | 15 | −65 $\rho<0,001$ | 19,5 | −54,5 $\rho<0,01$ | 34,9 | −18,6 | 0,4 | −99 $\rho<0,01$ |

Wirkung des Cimetidins und /+/ Nomifensins auf ein Modell von Wasser-Immersions-Stress-Geschwüren

Tabelle 4

| Kontrolle /−/ Nomifensin | | | /+/ Nomifensin | | Cimetidin und /+/ Nomifensin | | Cimetidin u. /+/ Nomifensin | |
|---|---|---|---|---|---|---|---|---|
| | 1 mg/kg | | 1 mg/kg | 20 mg/kg | | 1 mg/kg | 20 mg/kg | 2 mg/kg |
| | Ulcer-index | % der Unterdrückung des Ulcerindex | Ulcer-index | % der Unterdrückung des Ulcerindex | Ulcer-index | % der Unterdrückung des Ulcerindex | Ulcer-index | % der Unterdrückung des Ulcerindex |
| 1 | 2 | 3 | 4 | 5 | 6 | 7 | 8 | 9 |
| 56,95 (±11,4) | 50 (±17,1) | −13 | 16* (±5,3) | −72 | 9* (±5) | −84 | 6* (±5,5) | −89 |

* Es ist ein statistisch wesentlicher Unterschied gegenüber der Kontrollgruppe vorhanden.

**Patentansprüche**

1. Antiulcermittel auf der Basis von Histamin $H_2$-Blocker, dadurch gekennzeichnet, daß es als Antidepressant 4-Phenyl-2-methyl-8-amino-1,2,3,4-tetrahydroisochinolin der Formel 1

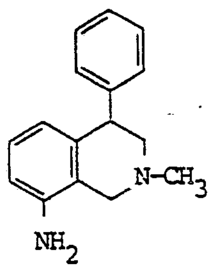

und/oder seine optisch aktiven Isomere oder seine physiologisch verträglichen Salze und gegebenenfalls ein spasmolytisches Mittel wie 4-(β-Piperidino-ethoxy)-2'-(methoxycarbonyl)-benzophenon enthält, wobei das Verhältnis des Histamin $H_2$-Blockers zum Antidepressant zwischen 160 : 1 und 2 : 1 liegt und das Verhältnis der Summe der beiden ersten zum krampflösenden Mittel von 425 : 1 bis 2,2 : 1 beträgt.

2. Mittel nach Anspruch 1, dadurch gekennzeichnet, daß die Histamin $H_2$-Blocker N-Cyano-N'-methyl-N''-[2-(4-methyl-5-imidazolyl)methylthio]-ethylguanidin oder (N-(2-[(5-Dimethylaminomethyl-2-furanyl)-methyl]-thioethyl)-N-methyl-2-nitro-1,1-ethendiamin) sind, der Antidepressant (+)4-Phenyl-2-methyl-8-amino-1,2,3,4-tetrahydroisochinolinsaures Maleat und das spasmolytische Mittel 4-(β-Piperidino-ethoxy)-2'-methoxycarbonyl-benzophenon ist und das Gewichtsverhältnis der Komponenten 10 : 1 : 0,2 beträgt.

3. Mittel nach Anspruch 2, dadurch gekennzeichnet, daß es N-Cyano-N'-methyl-N''-[2-(4-methyl-5-imidazolyl)-methylthio]-ethylguanidin oder (N-(2-[(5-Dimethylaminomethyl-2-furanyl)-methyl]-thioethyl)-N-methyl-2-nitro-1,1-ethendiamin) und (+)-4-Phenyl-2-methyl-8-amino-1,2,3,4-tetrahydroisochinolinsaures Maleat im Gewichtsverhältnis 10 : 0,5 enthält.

**Claims**

1. Anti-ulcer agent based on histamine $H_2$-blockers, characterised in that it contains, as anti-depressant, 4-phenyl-2-methyl-8-amino-1,2,3,4-tetrahydroisoquinoline of the formula 1

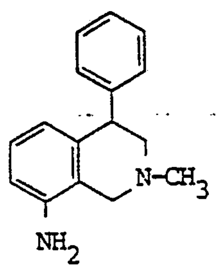

and/or its optically active isomers or its physiologically acceptable salts, and optionally a spasmolytic agent like 4-(β-piperidinoethoxy)-2'-(methoxycarbonyl)-benzophenone, wherein the ratio of the histamine $H_2$-blocker to the anti-depressant lies between 160 : 1 and 2 : 1 and the ratio of the sum of the first two to the spasm relieving agent amounts to 425 : 1 to 2.2 : 1.

2. Agent according to claim 1, characterised in that the histamine $H_2$-blockers are N-cyano-N'-methyl-N''-[2-(4-methyl-5-imidazolyl) methylthio]-ethylguanidine or (N-(2-[(5-dimethylaminomethyl-2-furanyl)-methyl]-thioethyl)-N-methyl-2-nitro-1,1-ethenediamine), the antidepressant is (+)-4-phenyl-2-methyl-8-amino-1,2,3,4-tetrahydroisoquinolinic acid maleate and the spasmolytic agent is 4-(β-piperidinoethoxy)-2'-methoxycarbonyl-benzophenone and the weight ratio of the components amounts to 10 : 1 : 0.2.

3. Agent according to claim 2, characterised in that it contains N-cyano-N'-methyl-N''-[2-(4-methyl-5-imidazolyl)-methylthio]-ethylguanidine or (N-(2-[(5-dimethylaminomethyl-2-furanyl)-methyl]-thioethyl)-N-methyl-2-nitro-1,1-ethenediamine) and (+)-4-phenyl-2-methyl-8-amino-1,2,3,4-tetrahydroisoquinolinic acid maleate in the weight ratio 10 : 0.5.

**Revendications**

1. Agent anti-ulcéreux à base de bloqueur $H_2$ de l'histamine, caractérisé par le fait qu'il renferme,

comme antidépresseur, de la phényl-4 méthyl-2 amino-8 tétrahydro-1,2,3,4 isoquinoléine de formule 1 :

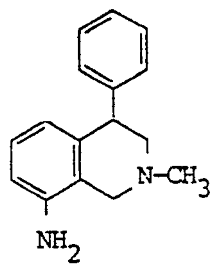

et/ou ses isomères optiquement actifs ou ses sels physiologiquement acceptables, et, le cas échéant, un agent spasmolytique comme la (β-pipéridinoéthoxy)-4 (méthoxycarbonyl)-2' benzophénone, le rapport du bloqueur $H_2$ de l'histamine à l'antidépresseur se situant entre 160 : 1 et 2 : 1, et le rapport de la somme des deux premiers à l'agent antispasmodique s'élevant à 425 : 1 à 2,2 : 1.

2. Agent selon la revendication 1, caractérisé par le fait que le bloqueur $H_2$ de l'histamine est la N-cyano-N'-méthyl-N"-[(méthyl-4 imidazolyl-5)-2 méthylthio]-éthylguanidine ou la (N-([(diméthylaminométhyl-5 furanyl-2)-méthyl]-2 thioéthyl)-N-méthyl-nitro-2 éthènediamine-1,1, l'antidépresseur est le maléate de l'acide (+)-phényl-4 méthyl-2 amino-8 tétrahydro-1,2,3,4 isoquinoléique, et l'agent spasmolytique est la (β-pipéridinoéthoxy)-4 méthoxycarbonyl-2' benzophénone, et le rapport pondéral des composants s'élève à 10 : 1 : 0,2.

3. Agent selon la revendication 2, caractérisé par le fait qu'il contient de la N-cyano-N'-méthyl-N"-[(méthyl-4 imidazolyl-5)-2 méthylthio]-éthylguanidine ou la (N-[(diméthylaminométhyl-5 furanyl-2-)-méthyl]-2 thioéthyl)-N-méthyl-nitro-2 éthènediamine-1,1, et le maléate de l'acide (+)-phényl-4 méthyl-2 amino-8 tétrahydro-1,2,3,4 isoquinoléique, dans un rapport pondéral de 10 : 0,5.